# EUROPEAN PATENT APPLICATION

(11) **EP 4 520 750 A1**
(43) Date of publication of application: **12.03.2025**
(21) Application number: 23823989.1
(22) Date of filing: 16.06.2023
(51) Int. Cl.: C07D 209/48, C07B 61/00

(54) **AMINO ACID HAVING FLUORINE AT ALPHA-POSITION, AND DERIVATIVE OF SAME**

(30) Priority: 17.06.2022 JP 2022097788
(71) Applicant: SynCrest Inc., Fujisawa-shi, Kanagawa 251-8555 (JP); University of The Ryukyus, Nakagami-gun, Okinawa 903-0213 (JP)
(72) Inventor: XU, Pengyu, Fujisawa-shi, Kanagawa 251-8555 (JP); YONEYAMA, Shin, Fujisawa-shi, Kanagawa 251-8555 (JP); ARIMITSU, Satoru, Nakagami-gun, Okinawa 903-0213 (JP); GENKA, Riko, Nakagami-gun, Okinawa 903-0213 (JP)
(74) Representative: Meissner Bolte Partnerschaft mbB
(86) International application number: PCT/JP2023/022346
(87) International publication number: WO 2023/243699

(57) **Abstract**

A first object of the present invention is to provide an amino acid compound having the α-position fluorinated by fluorinating the α-position with high yields. A second object of the present invention is to provide an amino acid compound having the α-position fluorinated by fluorinating the α-position with high enantioselectivity. Provided are amino acids having fluorine at the α-position and their derivatives.

## Description

### Technical Field

The present invention relates to an amino acid having a fluorine atom at the α-position and a derivative thereof.

### Background Art

Non-patent literature 1 discloses a method for synthesizing an amino acid having the α-position fluorinated by performing the Michael addition reaction using a nitro group.

### Citation List

### Non-patent Literature

NPL 1: Org. Biomol. Chem., 13, 2350-2359, 2015

### Summary of Invention

### Technical Problem

A first object of the present invention is to provide an amino acid compound having the α-position fluorinated by performing fluorination on the α-position with high yields.

A second object of the present invention is to provide an amino acid compound having the α-position fluorinated by performing fluorination on the α-position with high enantioselectivity.

### Solution to Problem

The present inventors conducted intensive research to achieve the objects above and found that the α-position of an amino acid compound can be fluorinated with high yields and high enantioselectivity by reacting an amino acid compound having the amine protected (Nphth group) with a phthaloyl group (Phth group) with a fluorinating agent, such as 1-chloromethyl-4-fluoro-1,4-diazoniabicyclo[2.2.2]octane bis(tetrafluoroborate), in an organic solvent in the presence of a catalyst.

The present inventors conducted further research based on this finding and completed the present invention.

Specifically, the present invention includes the following fluorinated amino acid compound and a production method for the fluorinated amino acid compound.

### Item 1

A fluorinated amino acid compound represented by the following formula (1): 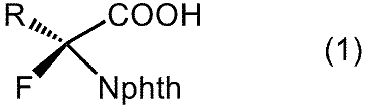 wherein
R represents an alkyl group, an alkoxy group, an aryl group, an ester group, an aralkyl group, an alkenyl group, or an alkynyl group, and
Nphth represents an N-phthaloyl group:

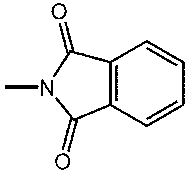

### Item 2

A method for producing a fluorinated amino acid compound represented by the following formula (2): 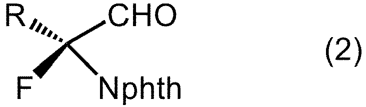 wherein
R represents an alkyl group, an alkoxy group, an aryl group, an ester group, an aralkyl group, an alkenyl group, or an alkynyl group, and
Nphth represents an N-phthaloyl group: 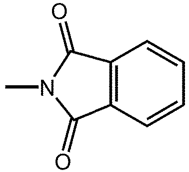
the method comprising mixing a compound represented by the following formula (3): 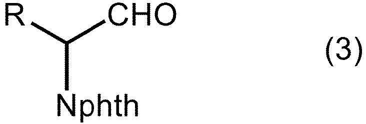 wherein R and Nphth are as defined in formula (1) above
with a fluorinating agent in an organic solvent in the presence of a catalyst to fluorinate the α-position of the compound represented by formula (3).

### Item 3

The method according to Item 2, wherein the organic solvent is at least one organic solvent selected from the group consisting of nitrile solvents, nitro solvents, alcohol solvents, halogen solvents, ether solvents, ester solvents, hydrocarbon solvents, and aromatic solvents.

### Item 4

The method according to Item 2 or 3, wherein the catalyst is an amine compound.

### Item 5

The method according to any one of Items 2 to 4, wherein the fluorinating agent is at least one fluorinating agent selected from the group consisting of N-fluorobenzenesulfonimide, 1-chloromethyl-4-fluoro-1,4-diazoniabicyclo[2.2.2]octane bis(tetrafluoroborate), 1-fluoro-2,4,6-trimethylpyridinium tetrafluoroborate, 2,6-dichloro-1-fluoropyridinium tetrafluoroborate, and 2,6-dichloro-1-fluoropyridinium triflate.

The compounds represented by formulas (1) and (2) can synthesize amino acid compounds having the α-position fluorinated.

The compound represented by formula (2) can improve the stability of the amino acid compound in the transition state by using an aldehyde compound, and can smoothly proceed with the fluorination of the α-position of the amino acid compound.

### Advantageous Effects of Invention

First, the present invention produces an amino acid compound having the α-position fluorinated by performing fluorination on the α-position with high yields.

Secondly, the present invention produces an amino acid compound having the α-position fluorinated by performing fluorination on the α-position with high enantioselectivity.

### Description of Embodiments

The present invention is described in detail below.

In the present specification, the terms "comprise" and "contain" include the concepts of comprising, consisting essentially of, and consisting of.

In the present specification, a numerical range indicated by "A to B" means "A or more and B or less."

### Method for Producing Fluorinated Amino Acid Compound

The method for producing an fluorinated amino acid compound of the present invention (a method for producing an amino acid compound having the α-position fluorinated (which is also referred to as "the production method of the present invention")) includes mixing an amino acid compound having the amine protected (Nphth group) with a phthaloyl group (Phth group) with a fluorinating agent in a liquid phase in the presence of a catalyst to allow fluorination of the α-position of the amino acid compound.

The production method of the present invention can stably produce an amino acid compound having the α-position fluorinated from an amino acid compound, which is a raw material compound, with high yields (74% to 99% yield) and with high enantioselectivity (75% ee to 95% ee).

### Amino acid compound (Substrate, Raw Material Compound)

The production method of the present invention includes fluorinating the α-position of an amino acid compound by using the amino acid compound (an amino acid compound having the amine protected (Nphth group) with a phthaloyl group (Phth group)) as a substrate.

The substrate is an amino acid compound having the structure represented by the following formula (3):

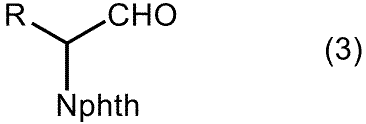

In formula (3), R is an alkyl group, an alkoxy group, an aryl group, an ester group, an aralkyl group, an alkenyl group, or an alkynyl group.

Nphth is an N-phthaloyl group.

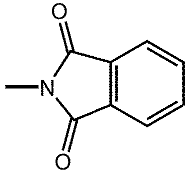

Using an aldehyde compound as a substrate ensures stability in the transition state and allows fluorination of the α-position of the amino acid compound to smoothly proceed.

In formula (3), R is an alkyl group, an alkoxy group, an aryl group, an ester group, an aralkyl group, an alkenyl group, an alkynyl group, or the like.

The alkyl group is preferably a linear, branched, or cyclic alkyl group. Specific examples include a C₁₋₄ linear or branched alkyl group, such as methyl, ethyl, n-propyl, isopropyl, n-butyl, isobutyl, sec-butyl, tert-butyl, and 1-ethylpropyl; a C₁₋₁₈ linear or branched alkyl group, such as n-pentyl, isopentyl, neopentyl, n-hexyl, isohexyl, 3-methylpentyl, n-heptyl, n-octyl, n-nonyl, n-decyl, n-undecyl, n-dodecyl, 5-propylnonyl, n-tridecyl, n-tetradecyl, n-pentadecyl, hexadecyl, heptadecyl, and octadecyl; a C₃₋₈ cyclic alkyl group, such as cyclopropyl, cyclobutyl, cyclopentyl, cyclohexyl, cycloheptyl, and cyclooctyl.

The alkoxy group is preferably a linear, branched, or cyclic alkoxy group. Specific examples include a C₁₋₄ linear or branched alkoxy group, such as methoxy, ethoxy, n-propoxy, isopropyloxy, n-butyloxy, isobutyloxy, sec-butyloxy, tert-butyloxy, and 1-ethylpropyloxy; a C₁₋₁₈ linear or branched alkoxy group, such as n-pentyloxy, isopentyloxy, neopentyloxy, n-hexyloxy, isohexyloxy, 3-methylpentyloxy, n-heptyloxy, n-octyloxy, n-nonyloxy, n-decyloxy, n-undecyloxy, n-dodecyloxy, 5-propylnonyloxy, n-tridecyloxy, n-tetradecyloxy, n-pentadecyloxy, hexadecyloxy, heptadecyloxy, and octadecyloxy; a C₃₋₈ cyclic alkoxy group, such as cyclopropyloxy, cyclobutyloxy, cyclopentyloxy, cyclohexyloxy, cycloheptyloxy, and cyclooctyloxy.

The aryl group is preferably phenyl, biphenyl, naphthyl, dihydroindenyl, 9H-fluorenyl, or the like.

The ester group is preferably a group with which the alkyl group described above is bonded via an ester bond. The ester group is also represented by an alkoxycarbonyl group, an alkoxycarbonylalkyl group, or a carboxyalkyl group. The ester group is preferably a methyl ester group (COOMe), an ethyl ester group (COOEt), or the like. The ester group is preferably a 3-carboxypropyl group ((CH₂)₃COOH), a 3-methyloxycarbonylpropyl group ((CH₂)₃COOMe), or the like.

The aralkyl group is preferably benzyl, phenethyl, trityl, 1-naphthylmethyl, 2-(1-naphthyl)ethyl, 2-(2-naphthyl)ethyl, or the like.

The alkenyl group is preferably an ethenyl group (vinyl group), a 2-propenyl group (allyl group), an isopropenyl group, a butenyl group, an isobutenyl group, a tert-butenyl group, a pentenyl group, a hexenyl group, a heptenyl group, an octynyl group, an isooctynyl group, a nonenyl group, or the like.

The alkynyl group is preferably an ethynyl group, a propynyl group, an isopropynyl group, a butynyl group, an isobutynyl group, a tert-butynyl group, a pentynyl group, a hexynyl group, an octynyl group, or a nonynyl group.

In formula (3), Nphth is an N-phthaloyl group.

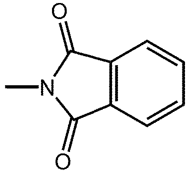

The phthaloyl group (Phth group) is a protecting group for an imide-based amine. Phthaloyl protection is performed by dehydration condensation of phthalic anhydride with an amine.

### Organic Solvent

The production method of the present invention includes fluorinating the α-position of an amino acid compound having the amine protected (Nphth group) with a phthaloyl group (Phth group) in an organic solvent.

The organic solvent is preferably at least one organic solvent selected from the group consisting of nitrile solvents (e.g., acetonitrile), nitro solvents (e.g., nitromethane), alcohol solvents (e.g., hexafluoroisopropanol (HFIP)), halogen solvents, ether solvents, ester solvents, hydrocarbon solvents, and aromatic solvents.

The nitrile solvents include acetonitrile and acrylonitrile.

The nitro solvents include nitromethane, nitroethane, and nitropropane.

The alcohol solvents include hexafluoroisopropanol (HFIP), trifluoromethanol, and pentafluoroethanol.

The halogen solvent is preferably dichloromethane (CH₂Cl₂), trichloromethane (CHCl₃), or the like.

The ether solvent is preferably tetrahydrofuran (THF), dioxane, diethyl ether (Et₂O), isopropyl ether (IPE), or the like.

The ester solvent is preferably ethyl acetate (AcOEt) or the like.

The hydrocarbon solvent is preferably n-hexane or the like.

The aromatic solvent is preferably toluene or the like.

The organic solvent is more preferably acetonitrile, nitromethane, or hexafluoroisopropanol (HFIP).

The amount of the organic solvent used is adjusted so that the concentration (M (mol/L)) of the amino acid compound having the structure represented by formula (3) (substrate) is preferably 0.005 M to 5 M, and more preferably 0.05 M to 2 M.

### Catalyst

The production method of the present invention includes fluorinating the α-position of an amino acid compound having the structure represented by formula (3) (substrate) in the presence of a catalyst.

The catalyst used in the production method of the present invention is preferably the following amine compounds.

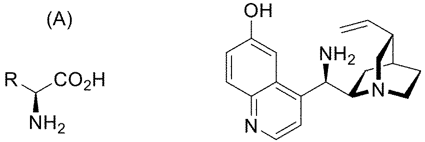

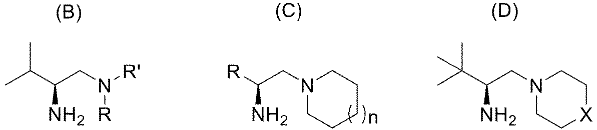

The catalyst used in the production method of the present invention is preferably the following amine compound.

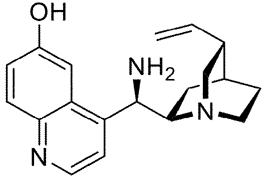

The catalyst used in the production method of the present invention is preferably an amine compound represented by the following formula (A):

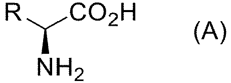

In formula (A), R is preferably an alkyl group, an aralkyl group, or the like.

The alkyl group is preferably a linear, branched, or cyclic alkyl group. Specific examples include C₁₋₄ linear or branched alkyl groups, such as methyl, ethyl, n-propyl, isopropyl, n-butyl, isobutyl, sec-butyl, tert-butyl, and 1-ethylpropyl; C₁₋₁₈ linear or branched alkyl groups, such as n-pentyl, isopentyl, neopentyl, n-hexyl, isohexyl, 3-methylpentyl, n-heptyl, n-octyl, n-nonyl, n-decyl, n-undecyl, n-dodecyl, 5-propylnonyl, n-tridecyl, n-tetradecyl, n-pentadecyl, hexadecyl, heptadecyl, and octadecyl; and C₃₋₈ cyclic alkyl groups, such as cyclopropyl, cyclobutyl, cyclopentyl, cyclohexyl, cycloheptyl, and cyclooctyl.

The aralkyl group is preferably benzyl, phenethyl, trityl, 1-naphthylmethyl, 2-(1-naphthyl)ethyl, 2-(2-naphthyl)ethyl, or the like.

In formula (A), R is preferably an alkyl group, such as methyl or tert-butyl, or an aralkyl group, such as benzyl.

The catalyst used in the production method of the present invention is preferably an amine compound represented by the following formula (B):

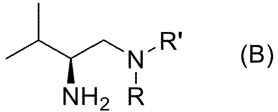

In formula (B), R and R' may be the same or different and are preferably each a hydrogen atom, an alkyl group, an aralkyl group, or the like.

The alkyl group is preferably a linear, branched, or cyclic alkyl group. Specific examples include C₁₋₄ linear or branched alkyl groups, such as methyl, ethyl, n-propyl, isopropyl, n-butyl, isobutyl, sec-butyl, tert-butyl, and 1-ethylpropyl; C₁₋₁₈ linear or branched alkyl groups, such as n-pentyl, isopentyl, neopentyl, n-hexyl, isohexyl, 3-methylpentyl, n-heptyl, n-octyl, n-nonyl, n-decyl, n-undecyl, n-dodecyl, 5-propylnonyl, n-tridecyl, n-tetradecyl, n-pentadecyl, hexadecyl, heptadecyl, and octadecyl; and C₃₋₈ cyclic alkyl groups, such as cyclopropyl, cyclobutyl, cyclopentyl, cyclohexyl, cycloheptyl, and cyclooctyl.

The aralkyl group is preferably benzyl, phenethyl, trityl, 1-naphthylmethyl, 2-(1-naphthyl)ethyl, 2-(2-naphthyl)ethyl, or the like.

In formula (B), R and R' may be the same or different and are preferably each a hydrogen atom, an alkyl group, such as ethyl or tert-butyl, or the like.

The catalyst used in the production method of the present invention is preferably an amine compound represented by the following formula (C):

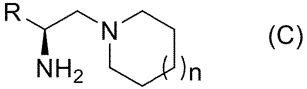

In formula (C), R is preferably an alkyl group, an aralkyl group, or the like.

The alkyl group is preferably a linear, branched, or cyclic alkyl group. Specific examples include C₁₋₄ linear or branched alkyl groups, such as methyl, ethyl, n-propyl, isopropyl, n-butyl, isobutyl, sec-butyl, tert-butyl, and 1-ethylpropyl; C₁₋₁₈ linear or branched alkyl groups, such as n-pentyl, isopentyl, neopentyl, n-hexyl, isohexyl, 3-methylpentyl, n-heptyl, n-octyl, n-nonyl, n-decyl, n-undecyl, n-dodecyl, 5-propylnonyl, n-tridecyl, n-tetradecyl, n-pentadecyl, hexadecyl, heptadecyl, and octadecyl; and C₃₋₈ cyclic alkyl groups, such as cyclopropyl, cyclobutyl, cyclopentyl, cyclohexyl, cycloheptyl, and cyclooctyl.

The aralkyl group is preferably benzyl, phenethyl, trityl, 1-naphthylmethyl, 2-(1-naphthyl)ethyl, 2-(2-naphthyl)ethyl, or the like.

In formula (A), R is preferably an alkyl group, such as methyl or isopropyl, an aralkyl group, such as benzyl, or the like.

In formula (C), n (the number of carbon atoms) is preferably an integer of 0, 1, or 2.

The catalyst used in the production method of the present invention is preferably an amine compound represented by the following formula (D):

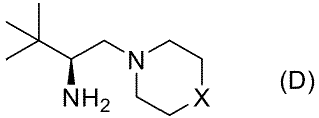

In formula (D), X is preferably a carbon atom, an oxygen atom, or the like.

In the production method of the present invention, at least one amine compound selected from the group consisting of the amine compounds described above is preferably used.

The amount of the catalyst used in the fluorination reaction is, in terms of molar ratio, preferably 1 mol% to 50 mol%, more preferably 10 mol% to 40 mol%, and even more preferably 10 mol% to 25 mol%, as a concentration relative to the substrate, that is, the amino acid compound having the structure represented by formula (3) (when the substrate is taken as 100 mol%).

In the production method of the present invention, using the catalyst described above in performing the fluorination reaction enables the production of an amino acid compound having the α-position fluorinated with high yields and with high enantioselectivity.

### Fluorinating Agent

The production method of the present invention includes mixing an amino acid compound having the structure represented by formula (3) (substrate) with a fluorinating agent to fluorinate the α-position of the amino acid compound having the structure represented by formula (3) (substrate).

The fluorinating agent is preferably at least one fluorinating agent selected from the group consisting of **N-**fluorobenzenesulfonimide (NFSI), 1-chloromethyl-4-fluoro-1,4-diazoniabicyclo[2.2.2]octane bis(tetrafluoroborate) (Selectfluor(R)), 1-fluoro-2,4,6-trimethylpyridinium tetrafluoroborate, 2,6-dichloro-1-fluoropyridinium tetrafluoroborate, and 2,6-dichloro-1-fluoropyridinium triflate; more preferably N-fluorobenzenesulfonimide (NFSI), and 1-chloromethyl-4-fluoro-1,4-diazoniabicyclo[2.2.2]octane bis(tetrafluoroborate); and particularly preferably 1-chloromethyl-4-fluoro-1,4-diazoniabicyclo[2.2.2]octane bis(tetrafluoroborate).

1-Chloromethyl-4-fluoro-1,4-diazoniabicyclo[2.2.2]octane bis(tetrafluoroborate) (Selectfluor(R)) has the following structure:

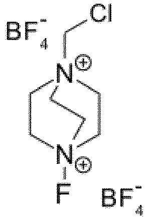

The amount of the catalyst used in the fluorination reaction **is,** in terms of molar ratio, preferably 0.1 to 20 equivalents, more preferably **0.9** to 5 equivalents, and even more preferably 1 to 3 equivalents relative to the substrate, that **is,** an amino acid compound having the structure represented by formula (3) (when the substrate is taken as 1 equivalent).

In the production method of the present invention, using the fluorinating agent described above in performing the fluorination reaction enables the production of an amino acid compound having the α-position fluorinated with high yields and with high enantioselectivity.

### Acidic Compound (e.g., TFA)

The production method of the present invention includes fluorinating the α-position of an amino acid compound having the structure represented by formula (3) (substrate) in the presence of an acidic compound **(e.g.,** TFA) catalyst.

The catalyst is preferably at least one acidic compound selected from the group consisting of trifluoroacetic acid (TFA), trifluoromethanesulfonic acid (TfOH), sulfuric acid (H₂SO₄), acetic acid (AcOH), and formic acid (HCOOH).

The amount of the acidic compound used in the fluorination reaction is, in terms of the amount used in an organic solvent in a molar ratio (when the entire organic solvent is taken as 100 mol%), preferably 1 mol% to 100 mol%, more preferably 5 mol% to 50 mol%, and even more preferably 10 mol% to 30 mol%.

In the production method of the present invention, using the acidic compound described above in performing the fluorination reaction enables the production of an amino acid compound having the α-position fluorinated with high yields and with high enantioselectivity.

### Step of Fluorinating α-Position of Amino Acid Compound (Substrate)

The production method of the present invention enables the production of a compound having the α-position fluorinated with high yields (74% to 99% yield) and with high enantioselectivity (75% ee to 95% ee) from an amino acid compound having the structure represented by formula (3) as a substrate (raw material compound).

The reaction temperature in the fluorination reaction step is preferably 0°C to 90°C, preferably 0°C to 60°C, and more preferably 10°C to 35°C (e.g., room temperature).

The reaction time in the fluorination reaction step is preferably 0.5 hours to 70 hours, preferably 10 hours to 60 hours, and more preferably 20 hours to 50 hours.

### Fluorinated Amino Acid Compound (Target Compound)

According to the production method of the present invention, a fluorinated amino acid compound having the structure represented by the following formula (2) can be obtained.

### Formula (2):

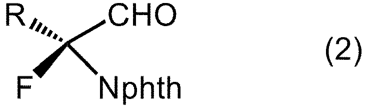

In formula (2), R represents an alkyl group, an alkoxy group, an aryl group, an ester group, an aralkyl group, an alkenyl group, or an alkynyl group; Nphth is an N-phthaloyl group:

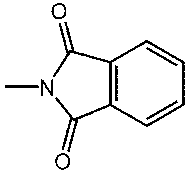

The present invention encompasses a fluorinated amino acid compound having the structure represented by the following formula (1). Oxidizing a fluorinated amino acid compound having the structure represented by formula (2) provides a fluorinated amino acid compound having the structure represented by the following formula (1).

A fluorinated amino acid compound represented by formula (1):

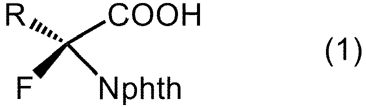

In formula (1), R is an alkyl group, an alkoxy group, an aryl group, an ester group, an aralkyl group, an alkenyl group, or an alkynyl group; Nphth is an N-phthaloyl group:

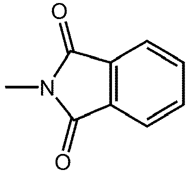

### Application Examples

An application example of the production method of the present invention is that diol compounds, aldol compounds, allyl compounds, etc. can be further produced with high enantioselectivity from the fluorinated amino acid compound prepared according to the production method of the present invention.

Embodiments of the present invention are described above; however, the present invention is not limited to these embodiments. Needless to say, the present invention can be performed in various forms without departing from the spirit and concept of the invention.

### Examples

Embodiments of the present invention are described in more detail below based on Examples.

The present invention is not limited to these Examples.

### Fluorination Reaction

As shown in Scheme 1 and Table 1, the α-position of an amino acid compound (substrate) was fluorinated.

Organic solvent: substrate concentration 0.5 M/various organic solvents
MeCN: acetonitrile
MeNO₂: nitromethane
HFIP/MeCN: hexafluoroisopropanol/acetonitrile
HFIP: hexafluoroisopropanol
Catalyst: various catalysts 20 mol%/substrate
Fluorinating agent: 1.5 equivalents/substrate
Fluorinating agent: 1-chloromethyl-4-fluoro-1,4-diazoniabicyclo[2.2.2]octane bis(tetrafluoroborate) (Selectfluor(R))
Acidic compound (additive): trifluoroacetic acid (TFA) 20 mol%/organic solvent
Reaction conditions: room temperature, 2 hours to 48 hours

A substrate, an amino acid compound having the structure represented by formula (3) (compound 1a), was fluorinated by using N-fluorobenzenesulfonimide (NFSI, compound F1) as a fluorinating agent (F⁺ reagent) in the presence of various catalysts and various organic solvents, thereby obtaining a target compound, which is a fluorinated amino acid compound having the structure represented by formula (2) (compound 2a).

The substrate is an amino acid compound having the structure represented by formula (3) in which R is a benzyl group (Ph-CH₂-, C₆H₅CH₂-).

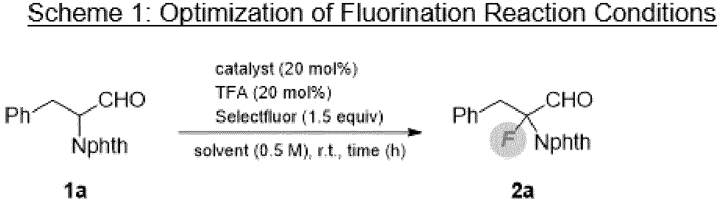

### Catalyst

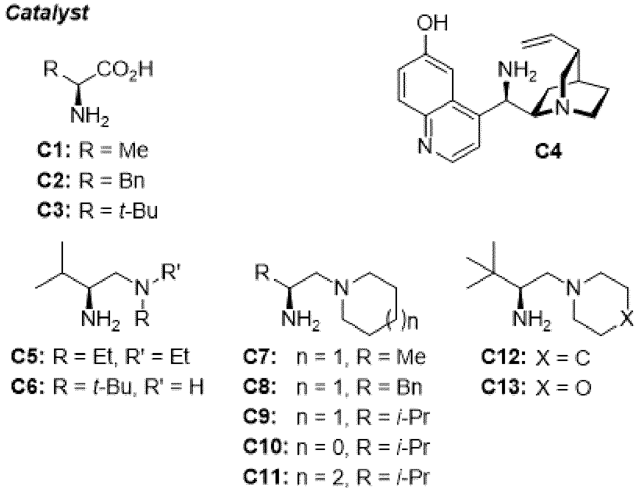

Me: **Methyl Group**
Et: Ethyl **Group**
i-Pr: Isopropyl **Group**
t-Bu: **Tert-Butyl Group**
Bn: Benzyl **Group**

### Example: Yield (%) (Catalysts C1 to C15)

The α-position of an amino acid compound (compound 1a) was fluorinated by using catalysts C1 to C13 in an organic solvent (MeCN, MeNO₂, HFIP/MeCN, or HFIP). The fluorination reaction of an amino acid compound (compound 1a) by using catalysts C1 to C13 enabled the production of a fluorinated amino acid compound (compound 2a) with high yields.

**Table 1**

| Table 1 | | | | |
|---|---|---|---|---|
| Example | Catalyst | Organic Solvent | Reaction Time (*a)(h) | Yield (%) (*b) |
| 1-1(*c) | C1 | MeCN | 22 | 81 |
| 1-2(*c) | C2 | MeCN | 24 | 93 |
| 1-3(*c) | C3 | MeCN | 22 | 76 |
| 1-4 | C4 | MeCN | 48 | 86 |
| 1-5 | C5 | MeCN | 2 | 86 |
| 1-6 | C6 | MeCN | 2 | 79 |
| 1-7 | C7 | MeCN | 3 | 94 |
| 1-8 | C8 | MeCN | 2 | 54 |
| 1-9 | C9 | MeCN | 4 | 80 |
| 1-10 | C10 | MeCN | 2 | 80 |
| 1-11 | C11 | MeCN | 3 | 90 |
| 1-12 | C12 | MeCN | 2 | 70 |
| 1-13 | C13 | MeCN | 3 | 92 |
| 1-14 | C13 | MeNO₂ | 2 | >99 |
| 1-15 | C13 | HFIP/MeCN(*d) | 7 | >99 |
| 1-16 | C13 | HFIP | 27 | 84 |

| | | | | |
|---|---|---|---|---|
| *a: The reaction time for the fluorination reaction was determined by monitoring the consumption of substrate compound (1a) using TLC. *b: The yield was determined based on ¹⁹F NMR of a crude reaction mixture using fluorobenzene as an internal standard. *c: In the absence of TFA *d: Examples 1-15, solvent HFIP/MeCN = 7/3 (v/v), substrate 0.3 M/organic solvent, TFA 30 mol%/organic solvent | | | | |

### Example: Enantioselectivity (%ee) (Solvent: MeNO₂, HFIP/MeCN, HFIP)

The α-position of an amino acid compound (compound 1a) was fluorinated using catalyst C13 in an organic solvent (MeNO₂, HFIP/MeCN, or HFIP). Fluorinating an amino acid compound (compound 1a) by using MeNO₂, HFIP/MeCN, or HFIP as an organic solvent and catalyst C13 enabled the production of a fluorinated amino acid compound (compound 2a) with high enantioselectivity.

**Table 2**

| Table 2 | | | | |
|---|---|---|---|---|
| Example | Catalyst | Organic Solvent | Reaction Time (*a)(h) | Enantioselectivity *ee* (%)(*b) |
| 2-1 | C13 | MeNO₂ | 2 | 78 |
| 2-2 | C13 | HFIP/MeCN(*c) | 7 | 95 |
| 2-3 | C13 | HFIP | 27 | 93 |

| | | | | |
|---|---|---|---|---|
| ***a:** The reaction time for the fluorination reaction was determined by monitoring the consumption of substrate compound (1a) using TLC. *b: After the Wittig reaction, enantioselectivity (ee%) was measured by HPLC equipped with Daicel Chiralpak OJ-3. *c: Example 2-2, solvent HFIP/MeCN = 7/3 (v/v), substrate 0.3 M/organic solvent, TFA 30 mol%/organic solvent | | | | |

### Scheme 2: Substrate Type

The range of substrates for the amino acid compound shown in scheme 2 was studied.

Organic solvent: substrate concentration 0.5 M/solvent HFIP/MeCN = 7/3 (v/v, ratio by volume)
Catalyst: catalyst C13, 20 mol%/substrate
Fluorinating agent: 1.5 equivalents/substrate
Fluorinating agent: 1-chloromethyl-4-fluoro-1,4-diazoniabicyclo[2.2.2]octane bis(tetrafluoroborate) (Selectfluor(R))
Acidic compound (additive): trifluoroacetic acid (TFA) 30 mol%/organic solvent
Reaction conditions: room temperature, 7 hours to 23 hours

The yield represents an isolated yield.

The reaction time of the fluorination reaction was measured by monitoring the consumption of β-diketone compound 1 by TLC.

The enantioselectivity (ee(%)) was determined using HPLC equipped with a chiral stationary phase.

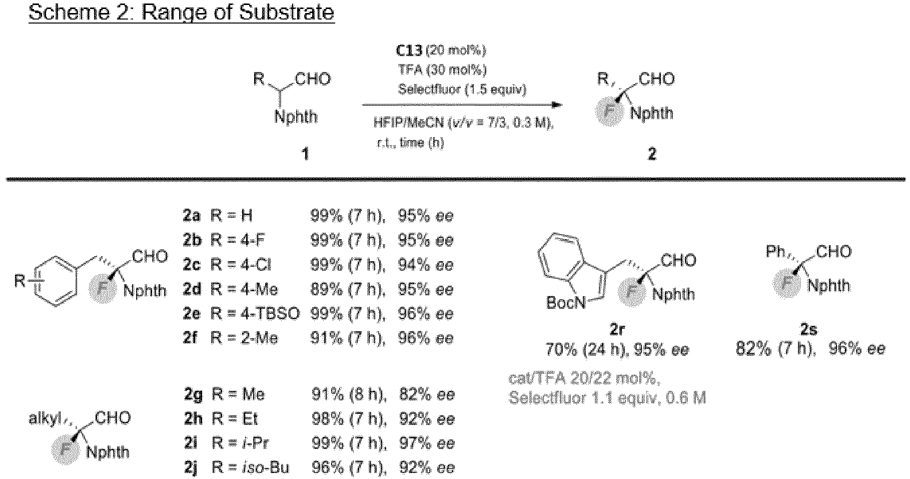

Even the use of the amino acid compounds represented by compounds 2a to 2s as substrates enabled the production of fluorinated amino acid compounds having the α-position fluorinated with high enantioselectivity with excellent yields.

### Scheme 3: Reference Example

A reference example (application in synthesis) of the fluorinated amino acid compounds produced by using the fluorination reaction of the present invention is shown below.

### Synthesis of α-fluorinated RG-108

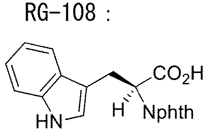

RG-108 is an inhibitor of DNA methyltransferase (N-phthalyl-L-tryptophan) and inhibits the enzyme with an IC₅₀ of 115 nM.

RG-108 has been shown to bind directly to the active site of the enzyme in molecular modeling.

RG-108 promotes the reprogramming of human and mouse somatic cells into iPS cells.

RG-108 has anticancer effects as an epigenomic drug.

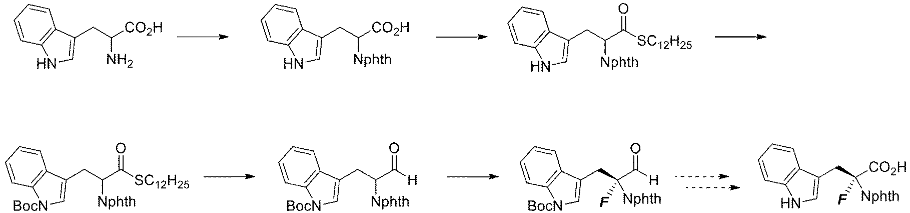

### Application in Dipeptides

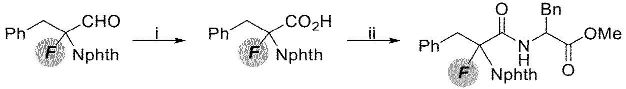

How to Proceed with Step:
(i) NaClO₂ (10 equiv), NaH₂PO₄ (8 equiv), 2-methyl-2-butene (30 equiv), t-BuOH/H₂O (v/v = 2/1, 0.05 M), r.t.
(ii) DIEP (3.0 equiv), HBTU (1.5 equiv), phenyl alanine methyl ester (1.1 equiv), THF (0.5 M), r.t.

Dipeptides were obtained as two diastereomers (17% + 37%).

### Industrial Applicability

The method for producing a fluorinated amino acid compound (an amino acid compound having the α-position fluorinated) of the present invention enables the fluorination reaction of an amino acid compound with high yields and with high enantioselectivity.

The fluorinated amino acid compounds obtained according to the production method of the present invention can be derived into special fluorinated amino acids, and are expected to have a wide range of industrial applications. The fluorinated amino acid compounds obtained according to the production method of the present invention can be used, for example, as a precursor in the synthesis of pharmaceuticals or peptides containing an amino acid having a fluorine atom at the α-position.

## Claims

1. A fluorinated amino acid compound represented by the following formula (1): 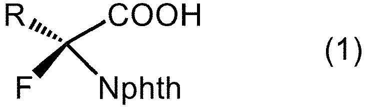 wherein
R represents an alkyl group, an alkoxy group, an aryl group, an ester group, an aralkyl group, an alkenyl group, or an alkynyl group, and
Nphth represents an N-phthaloyl group:

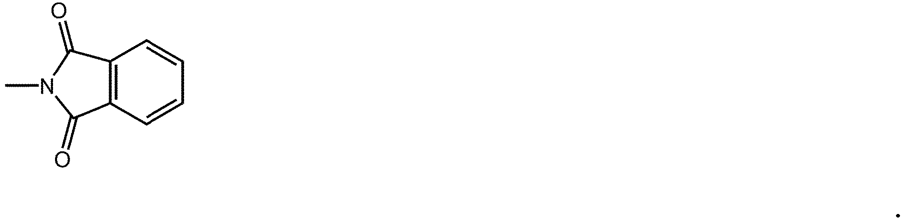

2. A method for producing a fluorinated amino acid compound represented by the following formula (2): 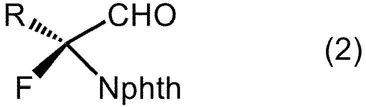 wherein
R represents an alkyl group, an alkoxy group, an aryl group, an ester group, an aralkyl group, an alkenyl group, or an alkynyl group, and
Nphth represents an N-phthaloyl group: 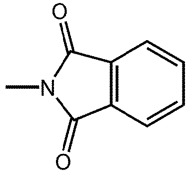
the method comprising mixing a compound represented by the following formula (3): 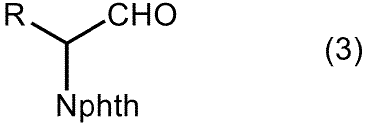 wherein R and Nphth are as defined in formula (1) above
with a fluorinating agent in an organic solvent in the presence of a catalyst to fluorinate the α-position of the compound represented by formula (3).

3. The method according to claim **2,** wherein the organic solvent is at least one organic solvent selected from the group consisting of nitrile solvents, nitro solvents, alcohol solvents, halogen solvents, ether solvents, ester solvents, hydrocarbon solvents, and aromatic solvents.

4. The method according to claim 2 or 3, wherein the catalyst is an amine compound.

5. The method according to any one of claims 2 to 4, wherein the fluorinating agent is at least one fluorinating agent selected from the group consisting of N-fluorobenzenesulfonimide, 1-chloromethyl-4-fluoro-1,4-diazoniabicyclo[2.2.2]octane bis(tetrafluoroborate), 1-fluoro-2,4,6-trimethylpyridinium tetrafluoroborate, 2,6-dichloro-1-fluoropyridinium tetrafluoroborate, and 2,6-dichloro-1-fluoropyridinium triflate.
